# EUROPÄISCHE PATENTSCHRIFT

(11) **EP 1 289 930 B1**
(45) Veröffentlichungstag und Bekanntmachung des Hinweises auf die Patenterteilung: **24.08.2005**
(21) Anmeldenummer: 01960298.6
(22) Anmeldetag: 12.06.2001
(51) Int. Cl.: C07C 227/04, C25B 3/04

(54) **VERFAHREN ZUR HERSTELLUNG VON 5-AMINOSALICYLSÄURE**
METHOD FOR PRODUCING 5-AMINOSALICYCLIC ACID
PROCEDE DE PREPARATION D'ACIDE 5-AMINOSALICYLIQUE

(30) Priorität: 15.06.2000 DE 10029410
(43) Veröffentlichungstag der Anmeldung: 12.03.2003
(73) Patentinhaber: Noveon IP Holdings Corp., Cleveland, OH 44141-3247 (US)
(72) Erfinder: SANCHEZ-CANO, Gaspar, I.D. Electroquimica, S.L., E-03113 Alicante (ES)
(74) Vertreter: Weber, Thomas
(86) Internationale Anmeldenummer: PCT/EP2001/006619
(87) Internationale Veröffentlichungsnummer: WO 2001/096280

(56) Entgegenhaltungen:
- EP-A- 0 253 788
- WO-A-86/03194

## Beschreibung

Die Erfindung betrifft ein neues Verfahren zur Herstellung von p-Aminophenolen, insbesondere von 5-Aminosalicylsäure, durch direkte elektrochemische Reduktion eines Sulfophenylazophenolderivats. Das Verfahren wird bevorzugt bei niedrigen Temperaturen und bevorzugt unter Verwendung einer speziellen Elektrode durchgeführt.

Bei p-Aminophenolen handelt es sich um technologisch wichtige Verbindungen, und insbesondere die 5-Aminosalicylsäure (5-ASA) der Formel 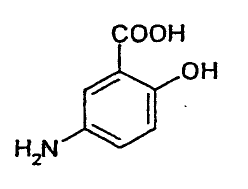 hat viele Anwendungen, beispielsweise in der Elektrophotographie, zur Herstellung von Färbungsmittel und Pigmenten und, vor allem in jüngerer Zeit, auch als Wirkstoff in der Medizin zur Behandlung einer Reihe von Erkrankungen. Verschiedene Verfahren zur Herstellung dieser Verbindungen und insbesondere von 5-ASA sind ebenfalls seit langem bekannt.

Technologisch besonders bedeutsam ist die Herstellung von 5-ASA durch Reduktion von 5-azoaromatischen Derivaten der Salicylsäure

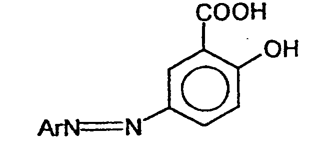

So beschreibt die EP-A 0 253 788 die Herstellung von 5-ASA im wesentlichen auf folgende Art und Weise

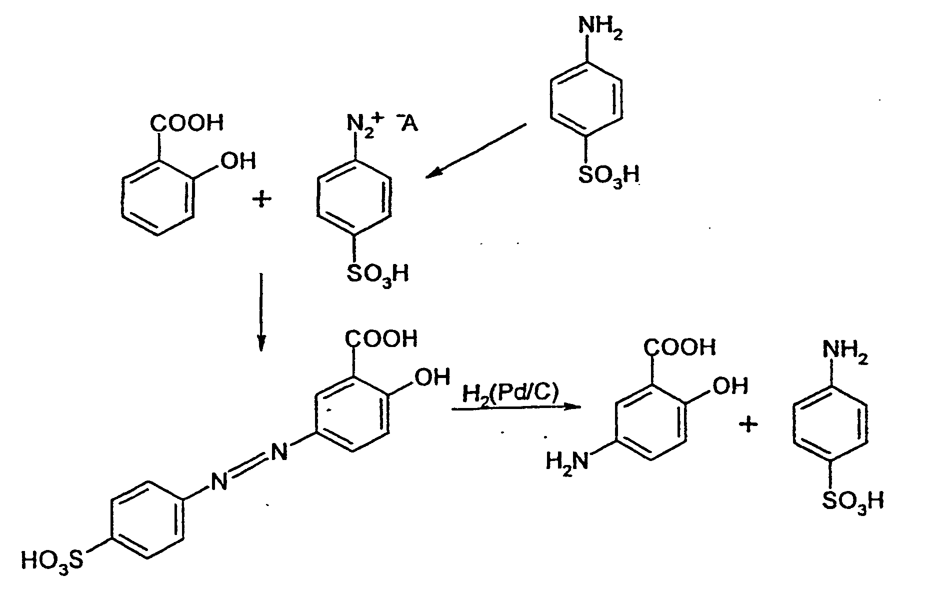

Hierbei wird zunächst Salicylsäure mit dem Diazosalz der Sulfanilsäure umgesetzt und die bei dieser Umsetzung gewonnene 5(para-Sulfophenylazo)salicylsäure wird dann durch katalytische Hydrierung zu 5-ASA umgesetzt. Die Hydrierung erfolgt mit Wasserstoffgas an einem Katalysator bei erhöhten Temperaturen von über 50°C.

Bei dieser Umsetzung ist vor allem nachteilig, daß mit Wasserstoffgas gearbeitet werden muß. Hydrierungen mit Wasserstoffgas sind zwar großtechnisch möglich, wegen der Explosionsgefahr sind derartige Verfahren aber unerwünscht und erfordern umfangreiche Sicherheitsmaßnahmen, was das Verfahren verteuert. Auch ist ein Arbeiten bei erhöhten Temperaturen aus wirtschaftlichen Gründen nicht vorteilhaft. Auch ist das Endprodukt bei der Hydrierung relativ stark verunreinigt und erfordert einen erhöhten Reinigungsaufwand.

Die WO 86/03194 beschreibt ein elektrochemisches Verfahren zur Herstellung verschiedener p-Aminophenole, beispielsweise auch von 5-ASA. Gelesen auf 5-ASA läuft das Verfahren im wesentlichen gemäß dem folgenden Reaktionsschema ab

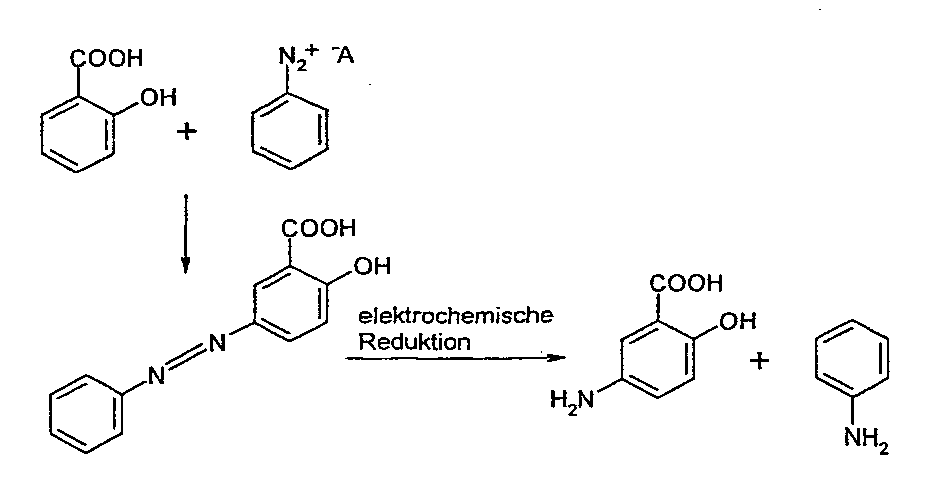

Das Verfahren muß bei einer Temperatur von über 50°C durchgeführt werden, und der bevorzugte Temperaturbereich liegt bei 70 bis 100°C.

Das Verfahren der WO 86/03194 weist den Nachteil auf, daß neben dem p-Aminophenol in gleichem Anteil Anilin entsteht, das als gesundheitsschädlich gilt. Insbesondere wenn die Zielverbindung für den medizinischen Einsatz vorgesehen ist, muß das entstandene Anilin soweit abgetrennt werden, daß die strengen gesetzlichen Grenzwerte eingehalten werden. Dies ist schwierig und mit hohen Kosten verbunden. Auch muß das Verfahren bei Temperaturen von deutlich über 50°C durchgeführt werden, was ebenfalls aus Kostengründen unerwünscht ist. Weiterhin wird aus den Beispielen der Druckschrift deutlich, daß die elektrochemische Umsetzung nicht vollständig ist, und nach der elektrochemischen Umsetzung erfolgt eine Vervollständigung der Umsetzung durch Zugabe von Natriumhydrosulfit. Die zugesetzten Mengen an Natriumhydrosulfit sind zu hoch, um ausschließlich zur Entfärbung des Umsetzungsprodukts zu dienen, wie es in der Druckschrift angegeben ist. Vielmehr ist offensichtlich, daß es hier auch darum geht, die unvollständig abgelaufene elektrochemische Reduktion durch chemische Reduktion mit Natriumhydrosulfit zu vervollständigen.

Aufgabe der vorliegenden Erfindung ist es daher, ein Verfahren zur Herstellung von p-Aminophenolen, insbesondere von 5-ASA, zur Verfügung zu stellen, das die Nachteile des Standes der Technik nicht aufweist und mit dem beispielsweise 5-ASA vorteilhafterweise und kostengünstig hergestellt werden kann. Die Umsetzung soll insbesondere auch bei tiefen Temperaturen durchgeführt werden, da dies die Nebenproduktbildung verringert.

Diese Aufgabe wird durch den Gegenstand der Patentansprüche gelöst. Die vorliegende Erfindung ist gerichtet auf ein

Verfahren zur Herstellung von p-Aminophenolen der Formel I 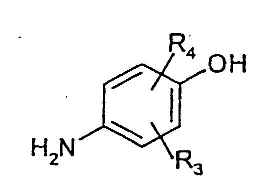 in der die Reste R₃ und R₄ unabhängig voneinander Wasserstoffatome, C₁-C₄-Alkylreste, Halogenatome, COOH-Gruppen, SO₃H-Gruppen oder NO₂-Gruppen darstellen,
durch elektrochemische Umsetzung, dadurch gekennzeichnet, daß eine Verbindung der Formel II 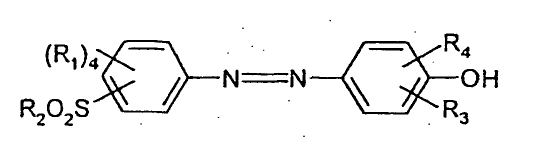 in der R₁ Wasserstoff, ein C₁-C₆-Alkylrest, eine Hydroxyl-, Sulfonyl- oder Aminogruppe oder ein Halogenatom sein kann, der Rest R₂ OR₅ oder NHR₅ sein kann, wobei R₅ Wasserstoff oder eine C₁-C₄-Alkylgruppe darstellen kann und die Reste R₁ gleich oder verschieden sein können und die Reste R₃ und R₄ wie vorstehend definiert sind, oder ein Salz davon, als Ausgangsverbindung für die elektrochemische Umsetzung verwendet wird und das Verfahren bei einer Temperatur von unter 50°C durchgeführt wird.

Bevorzugte Ausführungsformen sind aus den anhängenden Patentansprüchen ersichtlich.

Die Erfindung beruht auf dem überraschenden Befund, daß man Sulfonate der allgemeinen Formel 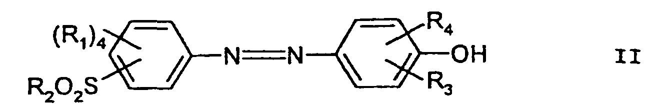 in der R₁ Wasserstoff, ein C₁-C₆-Alkylrest, eine Hydroxyl-oder Aminogruppe oder ein Halogenatom sein kann, der Rest R₂ OR₅ oder NHR₅ sein kann, wobei R₅ Wasserstoff oder eine C₁-C₄-Alkylgruppe darstellen kann und die Reste R₁ gleich oder verschieden sein können, die Reste R₃ und R₄ unabhängig voneinander Wasserstoffatome, C₁-C₄-Alkylreste, Halogenatome, COOH-Gruppen, SO₃H-Gruppen oder NO₂-Gruppen darstellen, oder deren Salze, insbesondere deren Alkalimetallsalze besonders vorteilhaft auf elektrochemischem Wege reduzieren kann, wobei bei Temperaturen von unter 50°C gearbeitet werden kann. Die hier als Nebenprodukt anfallenden Sulfanilprodukte sind im Gegensatz zu dem bei dem Verfahren der WO 86/03194 anfallenden Anilinprodukte nicht als giftig eingestuft, und einige werden als antibakterielle Mittel sogar therapeutisch eingesetzt. Eine so weitgehende Abtrennung des Nebenprodukts, wie dies bei der Entstehung von Anilinen erforderlich ist, ist daher bei dem erfindungsgemäßen Verfahren abhängig vom Einsatzzweck nicht notwendig. Die entstehenden Produkte können aber immer als Anilin-frei bezeichnet werden.

Erfindungsgemäß bevorzugt wird in dem elektrochemischen Verfahren zur Herstellung einer Verbindung der Formel I 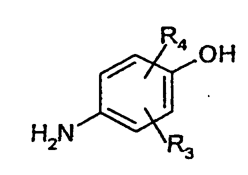 eine Verbindung der Formel II eingesetzt, in der zumindest drei Reste R₁ ein Wasserstoffatom darstellen. Am stärksten bevorzugt stellen alle vier Reste R₁ ein Wasserstoffatom dar. Ebenfalls bevorzugt sind Verbindungen der Formel II, in der die SO₂R₂-Gruppe in para-Stellung zur Azogruppe steht. Ebenfalls ist besonders bevorzugt, daß der Rest R₂ eine OH-Gruppe darstellt. Damit sind besonders bevorzugt Verbindungen, bei denen alle Reste R₁ ein Wasserstoffatom darstellen und der Rest -SO₂R₂ eine -SO₃H-Gruppe in p-Stellung zur Azo-Gruppe ist.

Besonders bevorzugt sind auch Verbindungen, in denen der Rest R₃ ein Wasserstoffatom darstellt. Bevorzugt sind ebenfalls Verbindungen, in denen der Rest R4 eine COOH-Gruppe darstellt, die wiederum bevorzugt in ortho-Stellung zur OH-Gruppe ist. Ebenfalls bevorzugt sind Salze dieser Verbindungen.

Damit wird als am meisten bevorzugte Verbindung der Formel II in dem erfindungsgemäßen Verfahren eine Verbindung der Formel 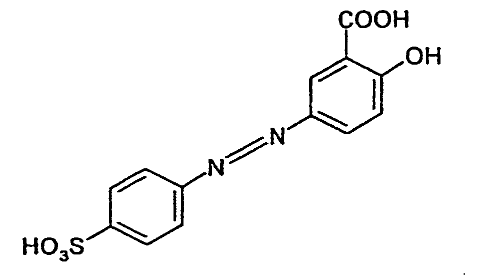 oder ein Salz davon eingesetzt.

Die Ausgangsverbindungen der Formel II können durch an sich bekannte Verfahren hergestellt werden, wie sie z.B. in der EP-A 0 253 788 prinzipiell beschrieben sind. Zur Herstellung der vorstehend genannten, besonders bevorzugten Verbindung der Formel II wird von Sulfanilsäure ausgegangen, während man zur Herstellung anderer Verbindungen der Formel II die entsprechenden Derivate der Sulfanilsäure bzw. die entsprechenden p-Aminophenole einsetzt.

Die elektrochemische Umsetzung der Verbindung der Formel II kann auf an sich bekannte Art und Weise erfolgen. Bevorzugt erfolgt die elektrochemische Umsetzung in einer Vorrichtung und unter Verwendung von Elektroden, wie sie in der EP-A 618 312 beschrieben werden, auf deren Inhalt insoweit Bezug genommen wird. Besonders bevorzugt erfolgt die elektrochemische Umsetzung ebenfalls in einer Vorrichtung und unter Verwendung von Elektroden, wie sie in der EP-A 778 360 beschrieben werden, auf deren Offenbarung insoweit Bezug genommen wird. Soweit in dieser Beschreibung nicht ausdrücklich anders angegeben, sind die in der EP-A 618 312 und EP-A 778 360 beschriebenen Vorrichtungen und Verfahrensbedingungen zur Durchführung der elektrochemischen Reduktion der Verbindung der Formel II zur Verbindung der Formel I bevorzugt.

Die elektrochemische Reduktion der Verbindung der Formel II erfolgt bevorzugt in einer Lösung, insbesondere in einer wäßrigen Lösung. Vorzugsweise sollte der pH-Wert der Lösung über 8 liegen, stärker bevorzugt über 9. In einer derart stark alkalischen Lösung wird die Verbindung der Formel II in dissoziierter Form vorliegen, so daß selbstverständlich auch anstelle der freien Säuren direkt die entsprechenden Salze, insbesondere die Alkalimetallsalze, eingesetzt werden können. Der pH-Wert der Lösung, die für die elektrochemische Reduktion eingesetzt wird, wird vorzugsweise durch Zusatz eines Alkalimetallhydroxids eingestellt. Gegebenenfalls können aber auch andere alkalisch reagierende Verbindungen eingesetzt werden.

Besonders bevorzugt wird die erfindungsgemäße elektrochemische Reduktion mit einer dreidimensionalen Kathode, insbesondere mit einer dreidimensionalen Karbon-Kathode, durchgeführt, die einen Metallkollektor aufweist. Derartige Kathoden sind in der EP-A 618 312 und der EP-A 778 360 beschrieben und auch kommerziell erhältlich. Bei einer dreidimensionalen Elektrode handelt es sich um eine poröse Elektrode, beispielsweise mit einer Struktur ähnlich der von Glaswolle oder Metallgeflecht, die dadurch eine große aktive Oberfläche aufweist.

Durch Verwendung derartiger Kathoden kann die reale Stromdichte aufgrund der großen Oberfläche niedrig gehalten werden, und man erzielt eine hohe Stromeffizienz für die elektrochemische Umwandlung. Chemische Reduktionsmittel, wie Natriumhydrosulfit, werden am Ende der Umsetzung zwar noch bevorzugt zur Entfärbung zugegeben, gegenüber dem aus der WO 86/03194 bekannten Verfahren kann ihre Menge aber erheblich verringert werden, da eine Vervollständigung der Umsetzung durch chemische Reduktion mit Natriumhydrosulfit nicht mehr notwendig ist.

Mit der erfindungsgemäß bevorzugten dreidimensionalen Kathode ist es auch möglich, während der gesamten Elektrolyse eine konstante Stromdichte beizubehalten.

Bei dem erfindungsgemäßen elektrochemischen Verfahren kann eine übliche Feld-Trennmembran verwendet werden. Besonders bevorzugt handelt es sich bei der Trennmembran um einen kationischen Ionenaustauscher, der bevorzugt perfluoriert ist. Derartige Feld-Trennmembranen sind kommerziell erhältlich. Auch hier kann zusätzlich wieder auf die in der EP-A 778 360 und der EP-A 618 312 offenbarten Feld-Trennmembranen verwiesen werden.

Die verwendbaren Anoden sind nicht besonders eingeschränkt, es sollten aber Anoden sein, die auch bei hohen pH-Werten zufriedenstellend arbeiten. Als Beispiel können Nickelanoden genannte werden. Auch hier kann zusätzlich wieder auf die in der EP-A 778 360 und der EP-A 618 312 offenbarten Anoden verwiesen werden.

Erfindungsgemäß ist die Stromdichte bevorzugt zwischen 500 und 2500 A/m² und ist bevorzugt während der gesamten Umsetzung konstant. Im übrigen kann auch hier wieder auf die in der EP-A 778 360 und der EP-A 618 312 offenbarten Stromdichten verwiesen werden.

Ein erheblicher Vorteil des erfindungsgemäßen Verfahrens liegt darin, daß die elektrochemische Reduktion bei niedrigen Temperaturen durchgeführt werden kann. Während das Verfahren gemäß der WO 86/03194 notwendigerweise Temperaturen von mehr als 50°C verlangt und in der Praxis sogar mehr als 70°C notwendig sind, wird das erfindungsgemäße Verfahren bevorzugt bei Temperaturen unter 50°C durchgeführt, stärker bevorzugt bei Temperaturen von 40°C oder darunter, insbesondere von 30°C oder darunter.

Die Reaktionszeiten sind von den einzelnen Parametern des elektrochemischen Verfahrens abhängig. Das Ende der Umsetzung kann von einem Fachmann durch übliche Verfahren, wie HPLC, leicht festgestellt werden. Nach Beendigung der elektrochemischen Reduktion kann die entstandene Verbindung der Formel I durch übliche chemische Methoden, wie sie im Stand der Technik bekannt sind, isoliert werden.

Das nachstehende Beispiel erläutert die Erfindung.

### Beispiel 1

### Herstellung von 5-ASA durch elektrochemische Reduktion von 5(para-Sulfophenylazo)salicylsäure:

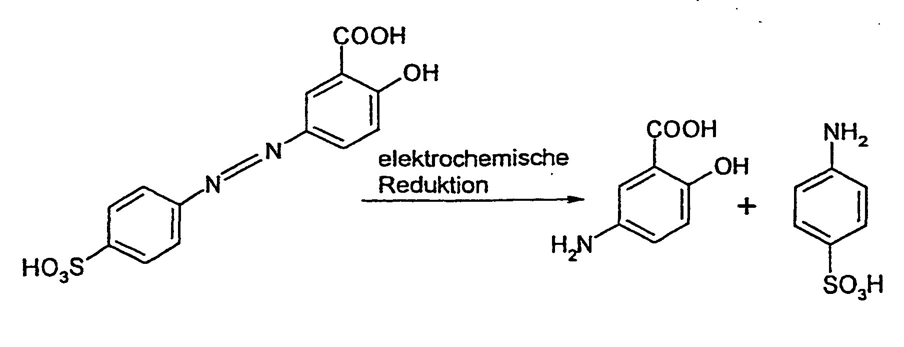

230 kg 5(p-Sulfophenylazo)salicylsäure (das nach "Grundlegende Operationen der Farbenchemie, 8, 5te Auflage, Wien 1983, Seite 150-151" hergestellt werden kann) und 85 kg Natriumhydroxid werden in 1500 1 Wasser gelöst. Die Lösung wird in einen Behälter gegeben, der mit einem elektrochemischen Reaktor ("REIM 330 der Firma I.D. Electroquimica S.L., Alicante, Spanien) in Kontakt steht. Die Lösung kann mit konstanter Geschwindigkeit durch den elektrochemischen Reaktor geführt werden und stellt die Kathodenflüssigkeit dar.

Der Reaktor besteht im wesentlichen aus einer kommerziell erhältlichen dreidimensionalen Karbon-Kathode mit hoher spezifischer Oberfläche mit einem Bleikollektor. Eine Karbon-Kathode mit Metallkollektor ist, unabhängig von den übrigen Parametern dieses speziellen Beispiels, die am stärksten bevorzugte Kathode der vorliegenden Erfindung. Als Metall kann, wie im vorliegenden Beispiel, Blei, aber auch Kupfer, Stahl oder Edelstahl verwendet werden. Als Anode wird eine Anode verwendet, die bei pH-Werten von 12 einsetzbar ist, beispielsweise eine Anode, die unter der Bezeichnung DSA-O₂ von der Firma PERMELEC erhältlich ist. Der elektrochemische Reaktor enthält weiterhin eine Feld-Trennmembran mit einem Arbeitsfeld von 4 m². Die Feld-Trennmembran ist kationisch und selektiv. Als Beispiel kann hier die von der Firma Dupont vertriebene Feld-Trennmembran mit der Bezeichnung NAFION 450 genannt werden.

Als Anodenflüssigkeit werden 900 1 einer Natriumhydroxidlösung verwendet, deren pH-Wert zwischen 10 und 11 liegt. Die Natriumhydroxidlösung wird in einen Behälter eingeführt, der mit dem elektrochemischen Reaktor verbunden ist.

Der pH-Wert der Anodenflüssigkeit wird während des Verfahrens durch kontrollierte Zugabe von 50%iger Natriumhydroxidlösung auf einem pH-Wert zwischen 10 und 11 konstant gehalten.

Während des Verfahrens wird eine Temperatur von 25°C bis 40°C sichergestellt. Die Kathodenflüssigkeit und die Anodenflüssigkeit werden mit einer Geschwindigkeit von 5000 l/Stunde durch den elektrochemischen Reaktor geführt. Die Stromzufuhr wird aktiviert mit einer mittleren Stromdichte von 1500 A/m². Das Verfahren wird 14 Stunden durchgeführt. Dabei wird insgesamt eine Ladung von 4,4 F pro Mol Ausgangsverbindung in Umlauf gebracht, was 110% der theoretischen stöchiometrischen Ladung entspricht.

Nach 14-stündiger Umsetzung ändert sich die Farbe der Lösung deutlich, was darauf hinweist, daß die Umsetzung abgeschlossen ist. Die Stromzufuhr wird unterbrochen, und die Lösung wird in einen üblichen Reaktor überführt, und 32%ige Salzsäurelösung wird bis zu einem pH-Wert von 3 bis 5 zugesetzt. 5-ASA fällt aus und wird abfiltriert. Nach Trocknen erhält man 90 kg 5-ASA mit einer Reinheit von mehr als 96% (nach HPLC). Die 5-ASA ist Anilin-frei.

Die Anodenflüssigkeit kann für eine weitere Umsetzung verwendet werden.

## Patentansprüche

1. Verfahren zur Herstellung von p-Aminophenolen der Formel I 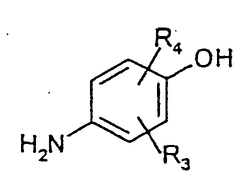 in der die Reste R₃ und R₄ unabhängig voneinander Wasserstoffatome, C₁-C₄-Alkylreste, Halogenatome, COOH-Gruppen, SO₃H-Gruppen oder NO₂-Gruppen darstellen,
durch elektrochemische Umsetzung, **dadurch gekennzeichnet, daß** eine Verbindung der Formel II 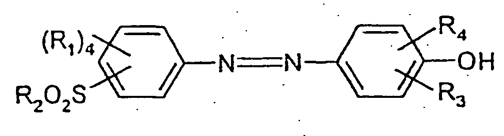 in der R₁ Wasserstoff, ein C₁-C₆-Alkylrest, eine Hydroxyl-oder Aminogruppe oder ein Halogenatom sein kann, der Rest R₂ OR₅ oder NHR₅ sein kann, wobei R₅ Wasserstoff oder eine C₁-C₄-Alkylgruppe darstellen kann und die Reste R₁ gleich oder verschieden sein können und die Reste R₃ und R₄ wie vorstehend definiert sind, oder ein Salz davon, als Ausgangsverbindung für die elektrochemische Umsetzung verwendet wird und das Verfahren bei einer Temperatur von unter 50°C durchgeführt wird.

2. Verfahren nach einem der vorstehenden Ansprüche, **dadurch gekennzeichnet, daß** das Verfahren bei einem pH-Wert von 7 oder mehr, vorzugsweise 9 oder mehr, durchgeführt wird.

3. Verfahren nach einem der vorstehenden Ansprüche, **dadurch gekennzeichnet, daß** in der Verbindung der Formel II alle Reste R₁ Wasserstoffatome darstellen.

4. Verfahren nach einem der vorstehenden Ansprüche, **dadurch gekennzeichnet, daß** in der Verbindung der Formel II der Rest SO₂R₂ eine Gruppe SO₃H in para-Stellung zur Azogruppe darstellt.

5. Verfahren nach einem der vorstehenden Ansprüche, **dadurch gekennzeichnet, daß** in der Verbindung der Formel II und der Verbindung der Formel I der Rest R₃ eine COOH-Gruppe in ortho-Stellung zur Hydroxylgruppe darstellt.

6. Verfahren nach Anspruch 5, **dadurch gekennzeichnet, daß** es sich bei der Verbindung der Formel I um 5-Aminosalicylsäure 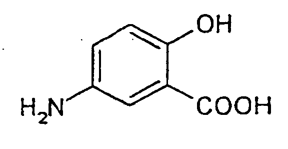 und bei der Verbindung der Formel II um die Verbindung 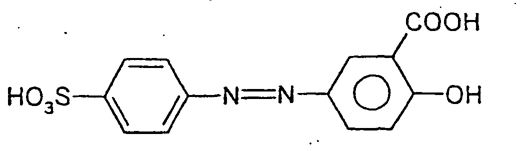 oder ein Salz davon handelt.

7. Verfahren nach einem der vorstehenden Ansprüche, **dadurch gekennzeichnet, daß** das Verfahren bei einer Temperatur von 40°C oder darunter, vorzugsweise bei 30°C oder darunter, durchgeführt wird.

8. Verfahren nach einem der vorstehenden Ansprüche, **dadurch gekennzeichnet, daß** in dem elektrochemischen Verfahren eine dreidimensionale Kathode verwendet wird.

9. Verfahren nach Anspruch 8, **dadurch gekennzeichnet, daß** es sich bei der dreidimensionalen Kathode um eine Karbon-Kathode mit Metallkollektor, vorzugsweise mit Bleikollektor, handelt.

## Claims

1. A process for the preparation of p-aminophenols of formula I 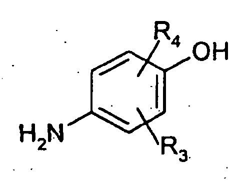 in which the residues R₃ and R₄ independently represent hydrogen atoms, C₁-C₄ alkyl residues, halogen atoms, COOH groups, SO₃H groups or NO₂ groups, by an electrochemical reaction, **characterized in that** a compound of formula II 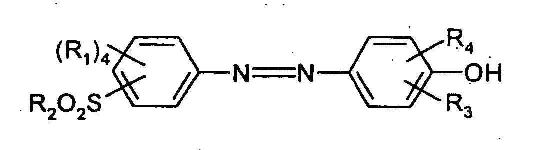 in which R₁ may be hydrogen, a C₁-C₆ alkyl residue, a hydroxy or amino group, or a halogen atom, the residue R₂ may be OR₅ or NHR₅, wherein R₅ may represent hydrogen or a C₁-C₄ alkyl group, and the residues R₁ may be the same or different, and the residues R₃ and R₄ are as defined above, or a salt thereof is used as the starting compound for said electrochemical reaction, and the process is performed at a temperature of below 50°C.

2. The process according to any of the preceding claims, **characterized in that** the process is performed at a pH value of 7 or greater, preferably 9 or greater.

3. The process according to any of the preceding claims, **characterized in that** all residues R₁ in the compound of formula II represent hydrogen atoms.

4. The process according to any of the preceding claims, **characterized in that** the residue SO₂R₂ in the compound of formula II represents an -SO₃H group in a para position with respect to the azo group.

5. The process according to any of the preceding claims, **characterized in that** the residue R₃ in the compound of formula II and in the compound of formula I represents a COOH group in an ortho position with respect to the hydroxy group.

6. The process according to claim 5, **characterized in that** the compound of formula I is 5-aminosalicylic acid 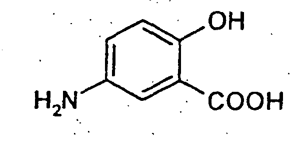 and the compound of formula II is the compound 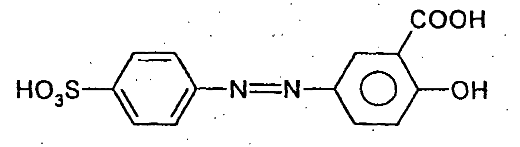 or a salt thereof.

7. The process according to any of the preceding claims, **characterized in that** the process is performed at a temperature of 40 °C or below, preferably 30 °C or below.

8. The process according to any of the preceding claims, **characterized in that** a three-dimensional cathode is used in said electrochemical process.

9. The process according to claim 8, **characterized in that** the said three-dimensional cathode is a carbon cathode, comprising a metal collector, preferably comprising a lead collector.

## Revendications

1. Procédé pour la préparation de p-aminophénols de formule I 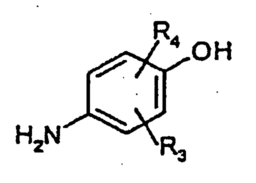 dans laquelle les radicaux R₃ et R₄ représentent indépendamment l'un de l'autre des atomes d'hydrogène, des radicaux alkyle en C₁ à C₄, des atomes d'halogène, des groupes COOH, des groupes SO₃H ou des groupes NO₂,
par réaction électrochimique, **caractérisé en ce que** l'on utilise comme composé de départ pour la réaction électrochimique un composé de formule II 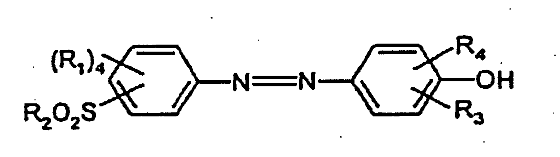 dans laquelle R₁ peut être un hydrogène, un radical alkyle en C₁ à C₆, un groupe hydroxyle ou amino ou un atome d'halogène, le radical R₂ peut être OR₅ ou NHR₅, R₅ pouvant représenter un hydrogène ou un groupe alkyle en C₁ à C₄ et les radicaux R₁ pouvant être identiques ou différents et les radicaux R₃ et R₄ étant définis comme précédemment, ou un sel de celui-ci, et **en ce que** l'on réalise le procédé à une température inférieure à 50 °C.

2. Procédé selon l'une des revendications précédentes, **caractérisé en ce que** l'on réalise le procédé à une valeur de pH de 7 ou plus, de préférence de 9 ou plus.

3. Procédé selon l'une des revendications précédentes, **caractérisé en ce que**, dans le composé de formule II, tous les radicaux R₁ représentent des atomes d'hydrogène.

4. Procédé selon l'une des revendications précédentes, **caractérisé en ce que**, dans le composé de formule II, le radical SO₃R₂ représente un groupe SO₃H en position para par rapport au groupe azo.

5. Procédé selon l'une des revendications précédentes, **caractérisé en ce que**, dans le composé de formule II et le composé de formule I, le radical R₃ représente un groupe COOH en position ortho par rapport au groupe hydroxyle.

6. Procédé selon la revendication 5, **caractérisé en ce qu'**il s'agit pour le composé de formule I de l'acide 5-aminosalicylique 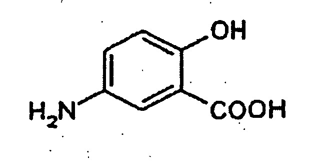 et pour le composé de formule II, du composé 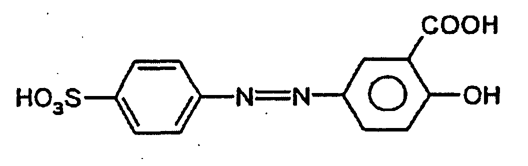 ou d'un sel de ceux-ci.

7. Procédé selon l'une des revendications précédentes, **caractérisé en ce que** l'on réalise le procédé à une température de 40 °C ou moins, de préférence de 30 °C ou moins.

8. Procédé selon l'une des revendications précédentes, **caractérisé en ce que** l'on utilise dans le procédé électrochimique une cathode tridimensionnelle.

9. Procédé selon la revendication 8, **caractérisé en ce qu'**il s'agit pour la cathode tridimensionnelle d'une cathode de carbone avec un collecteur de métal, de préférence avec un collecteur de plomb.
